**Europäisches Patentamt**

**European Patent Office**

**Office européen des brevets**

(19)

(11) Veröffentlichungsnummer: **0 079 486**
**B1**

(12) # EUROPÄISCHE PATENTSCHRIFT

(45) Veröffentlichungstag der Patentschrift:
14.01.87

(51) Int. Cl.⁴: **A 61 M 25/00**

(21) Anmeldenummer: 82109782.1

(22) Anmeldetag: 22.10.82

(54) Versteifungskern für einen Katheterschlauch.

(30) Priorität: 10.11.81 DE 8132839 U

(73) Patentinhaber: Intermedicat GmbH,
Gerliswilstrasse 45, CH- 6020 Emmenbrücke (CH)

(43) Veröffentlichungstag der Anmeldung:
25.05.83 Patentblatt 83/21

(72) Erfinder: Herlitze, Gerhard, Binsdorfer Strasse 4,
D-3507 Baunatal 7 (DE)

(45) Bekanntmachung des Hinweises auf die Patenterteilung:
14.01.87 Patentblatt 87/3

(74) Vertreter: von Kreisler, Alek, Dipl.- Chem.,
Deichmannhaus am Hauptbahnhof, D-5000 Köln 1
(DE)

(84) Benannte Vertragsstaaten:
AT BE CH DE FR GB IT LI LU NL SE

(56) Entgegenhaltungen:
EP-A-0 014 424
DE-A-2 542 241
US-A-2 856 934
US-A-3 731 376
US-A-3 973 556
US-A-3 996 939
US-A-4 257 421

## Beschreibung

Die Erfindung bezieht sich auf ein Katheterbesteck, bestehend aus einem flexiblen Katheterschlauch mit zylindrischem Hohlraum und einem stabförmigen Versteifungskern, bei dem mindestens eine reißfeste Seele aus mehreren Drähten in eine Umhüllung aus Kunststoff eingebettet ist und der aus dem Hohlraum des Katheterschlauches herausziehbar ist.

Langzeittherapien von Infusionslösungen werden vorzugsweise über Venenkatheter eingeleitet. Es sind verschiedene Ausführungsformen von Venenkathetern bekannt, die sich durch Länge, Durchmesser und Anwendungstechnik unterscheiden. Meistens werden Venenkatheter ohne Zuhilfenahme des Röntgenschirmes gelegt, wobei allerdings eine nachträgliche Lagekontrolle durchgeführt wird.

In manchen Fällen, zum Beispiel bei der Verlegung eines zum Herzen führenden Katheters, müssen der Vorschub und die Lage des Katheterschlauches jedoch am Röntgenschirm überwacht werden. Zur Darstellung des Katheterschlauches am Röntgenschirm wird bisher entweder der Katheterschlauch selbst mit Röntgenkontraststreifen versehen bzw. vollpigmentiert hergestellt und angefertigt oder es wird der aus einem röntgenkontrastmittelhaltigen Kunststoff bestehende, den Hohlraum des Katheterschlauches ausfüllende Versteifungskern zur Verbesserung der Darstellung am Röntgenschirm benutzt. Die den verschiedenen Arten von Kunststoffen beigemengten Röntgenkontrastmittel verringern die Festigkeit des Ausgangsmaterials und beeinträchtigen je nach prozentualem Anteil die Zugfestigkeit erheblich. Dies kann zu Abrissen des Versteifungskerns führen, die für den Patienten gefährlich sind.

Ein weiterer Mangel der bekannten Versteifungskerne aus Kunststoff besteht darin, daß die gewünschte Steifigkeit und Rückstellfähigkeit nur in einem kleinen Bereich variiert werden können. Aufgrund ihrer physikalischen Eigenschaften sind diese Versteifungskerne auch in keiner Weise geeignet, als Leitschiene für einen in Körperhöhlen zu plazierenden Venenkatheter zu dienen, wie es bei der sogenannten Seldinger-Methode praktiziert wird. Bei der Seldinger-Methode werden zwar gut funktionierende, schraubenförmig gewickelte Drähte als Versteifungskern für einen Katheterschlauch benutzt, die jedoch aufgrund ihres aufwendigen Aufbaus als Einmalartikel ausgesprochen teuer sind.

US-A-2 856 934 beschreibt eine Katheterführung für ein starres Katheterrohr, das an einem Ende gekrümmt und verjüngt ist. Die Katheterführung besteht aus einem zentralen gewebten Kern aus Nylonfäden, der außen mit Kunststoff beschichtet ist und innen einen einzigen feinen Metalldraht, vorzugsweise Silberdraht, enthält. Federnd rückstellfähig ist dieser Aufbau nicht. In einem Bereich zwischen ihren Enden ist die Katheterführung verdickt ausgebildet, so daß ein Anschlag für das aufgeschobene Katheterrohr entsteht. Zwischen der Katheterführung und der Innenwand des Katheterrohres ist ein Ringspalt vorhanden, durch den Körperfluid gegen das körperferne Ende des Katheterrohres zu einem Auslaß fließen soll. Eine solche Katheterführung ist nicht geeignet, um einen flexiblen Katheterschlauch bei der Überwindung von Krümmungen und Verengungen im Körper zu führen und zu plazieren. Damit wird für diesen Einsatzzweck der Vorteil einer durch die Seele möglicherweise gesteigerten Zerreißfestigkeit der Katheterführung praktisch bedeutungslos.

Ferner ist ein elektrisch leitfähiger Katheter bekannt (US-A-3 731 376), der eine verseilte Metalldrahtlitze aufweist, die mit einer Kunststoffumhüllung umgeben ist. Die Umhüllung besteht aus einem Schlauch, der auf die Litze stramm aufgezogen und an ihren Enden mit ihr fest verbunden ist. Metalldrahtlitze und Umhüllung bilden eine untrennbare Einheit, die als Herzschrittmacherelektrode in einen menschlichen Körper eingeführt wird und in diesem verbleibt. Diese bekannte Anordnung dient nicht als herausziehbarer Versteifungskern für einen flexiblen Katheterschlauch und sie wäre für diesen Zweck auch nicht geeignet, weil die Metalldrahtlitze nur im Bereich ihrer Enden in das Material der Umhüllung eingebettet ist, während der größte Teil ihrer Länge von der Umhüllung zwar stramm umgeben, jedoch nicht in diese eingebettet ist. Damit besteht die Möglichkeit der Verdrehung und Verschiebung der Umhüllung auf der Metalldrahtlitze und dies könnte Verformungen auf der Außenfläche der Umhüllung zur Folge haben, die die Verschiebung der Anordnung in einem flexiblen Katheterschlauch behindern oder sogar verhindern könnten. Dies wäre bei der Verlegung eines flexiblen Katheterschlauches mit Versteifungskern sehr gefährlich, weil bei den Manipulationen zum Herausziehen des Versteifungskerns aus dem Katheterschlauch seine Position verändert und die Genauigkeit der Katheterverlegung in Frage gestellt wird. Würde man, um einer solchen Behinderung des Herausziehens vorzubeugen, den Außendurchmesser der Metalldrahtlitze-Umhüllung-Anordnung in bezug auf den Innendurchmesser des Katheterschlauches ausreichend klein bemessen, so würde der Versteifungs- und Führungseffekt für den Katheterschlauch beeinträchtigt und außerdem würde durch den vergrößerten Ringspalt zwischen der Einlage und dem Katheterschlauch Körperflüssigkeit nach außen dringen, was vermieden werden soll.

Einen aus dem zylindrischen Hohlraum eines flexiblen Katheterschlauches herausziehbaren

stabförmigen Versteifungskern mit einer reißfesten Seele aus mehreren Drähten, die in eine Umhüllung aus Kunststoff eingebettet sind, beschreibt DE-A-2 542 241. Hierbei sind aber die Drähte in der Umhüllung so ausgebildet und angeordnet, daß der Versteifungskern von Hand in eine bleibende Form verbiegbar ist. Diese bleibende Gestaltsveränderung des Versteifungskerns durch Biegen ist bei der Verlegung flexibler Katheterschläuche, beispielsweise in der Vene, gänzlich unzweckmäßig, weil hierbei der Versteifungskern mit dem flexiblen Katheterschlauch den Krümmungen und Verengungen der Blutgefäße weich und flexibel folgen muß, um in die Zielzone für den Katheterschlauch zu kommen.

Der Erfindung liegt die Aufgabe zugrunde, ein preiswertes Katheterbesteck zu schaffen, dessen röntgenfähiger, reißfester Versteifungskern flexibel rückstellfähig ist und ohne Körperfluidrückfluß durch den Katheterschlauch dessen vorsichtig geführte Einführung in das Zielgebiet ermöglicht.

Diese Aufgabe wird bei einem Katheterbesteck der eingangs erwähnten Art dadurch gelöst, daß die reißfeste Seele als rückstellfähige Litze aus geflochtenen oder gezwirnten Stahldrähten ausgebildet ist und daß der Versteifungskern den Hohlraum des Katheterschlauches spaltfrei ausfüllt.

Die kunststoffummantelte Litze aus Stahldrähten verleiht dem Versteifungskern für einen Katheterschlauch hohe Reißfestigkeit und gute federnde Rückstellfähigkeit, durch die er in der Lage ist, den Katheterschlauch durch Krümmungen und Biegungen hindurch zuverlässig in die gewünschte Position zu bringen. Durch Änderung der Stahlqualität und der Stahldrahtdurchmesser lassen sich unterschiedliche Funktionsverhalten erzielen, die eine Anpassung des Versteifungskerns an den jeweiligen Verwendungszweck ermöglichen. Der erfindungsgemäße Versteifungskern macht den Vorgang des Katheterisierens sicherer, weil er nicht abreißt und seine federnde Rückstellfähigkeit ihm Führungseigenschaften verleiht, die Fehlplazierungen eines Katheterschlauches verhindern.

Die mindestens eine geflochtene oder gezwirnte multifile Seele verleiht dem sie ummantelnden Kunststoffstrang hohe Zugfestigkeit, so daß es möglich ist, ohne die Gefahr des Abreißens des Versteifungskerns Kunststoffe mit erhöhtem Röntgenkontrastmittelanteil zu verwenden. Auf diese Weise wird der Katheterschlauch, dessen Totraum von dem Versteifungskern ausgefüllt wird, am Röntgenschirm sehr gut sichtbar und seine Bewegung und Lage lassen sich zuverlässig überwachen. Da die Litze selbst aus röntgenologisch sichtbarem Material hergestellt ist, ergänzt sie den Effekt des Röntgenkontrastmittels im Kunststoff oder ersetzt dieses, wenn Kunststoff ohne Röntgenkontrastmittelzugabe zur Herstellung des Kunststoffstranges verwendet worden ist. Der Versteifungskern zeichnet sich durch zweckmäige Steifigkeit und gute Rückstellfähigkeit aus. Beide Faktoren können durch Wahl des Durchmessers und der Anzahl der Einzelfäden einer Litze in weitem Bereich variiert und auf die gewünschte Ausführung eingestellt werden. Durch die Kunststoffummantelung entsteht eine geglättete Oberfläche, die mit der Auswahl eines geeigneten Werkstoffes, zum Beispiel Polyethylen, Polytetrafluorethylen oder Polyurethan der Blutkoagulation entgegenwirkt.

In vorteilhafter Ausgestaltung der Erfindung ist die Litze in der Umhüllung konzentrisch angeordnet.

Die Stahldrähte der Litze können gleiche oder zueinander verschiedene Stärke aufweisen. Auch auf diese Weise lassen sich die physikalischen Eigenschaften des Versteifungskerns beliebig beeinflussen.

Die Litze kann im Linksschlag oder im Rechtsschlag gefertigt sein.

Die Umhüllung kann aus verschiedenen Kunststoffen bestehen oder aus nur einem einzigen Kunststoffmaterial hergestellt sein.

Bei Einsatz des Versteifungskerns als Leitschiene für einen in Körperhöhlen einzuschiebenden Katheter ist vorteilhafterweise mit dem Einführende der Umhüllung eine hochflexible Spitze aus Kunststoff fest verbunden. Die Spitze kann massiv oder hohl als Rohr ausgebildet und in geeignetem Schweißverfahren mit der Kunststoffummantelung der Litze verbunden sein. In dieser Ausfürung stellt der Vertsteifungskern für einen Katheterschlauch ein zum Einmalgebrauch geeignetes preiswertes Führungselement zur Plazierung und Lagekorrektur von Kathetern dar. Mit diesem Versteifungskern mit definierter Steifigkeit, der eine Spitze hoher Flexibilität aufweist, können sonst nicht passierbare Hindernisse im Verlauf gekrümmter Körperhöhlen, insbesondere der Vene, überwunden und problematische Fehllagen korrigiert werden, wobei eine exakte Röntgenkontrolle möglich ist.

In der Zeichnung ist ein Ausführungsbeispiel der Erfindung schematisch dargestellt. Es zeigt:.

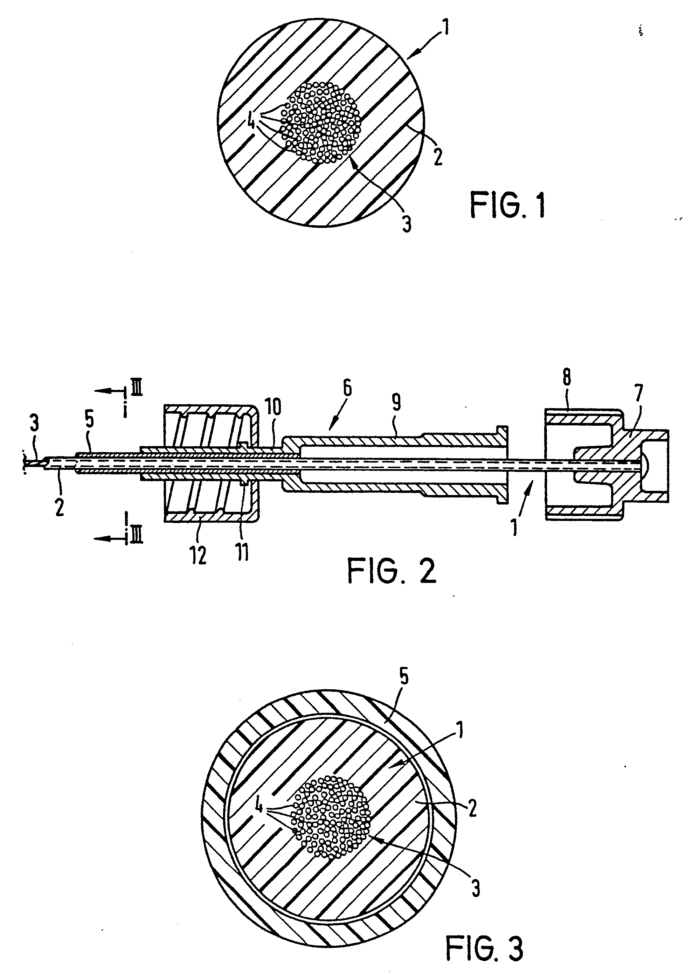

Figur 1 einen Querschnitt des Versteifungskerns

Figur 2 einen Längsschnitt eines Katheters mit Versteifungskern und

Figur 3 einen Querschnitt längs der Linie 3-3 in Figur 2.

Der Versteifungskern 1 zur Ausfüllung des Totraumes eines Katheterschlauches S, insbesondere eines Venenkatheters 6, besteht aus einem Kunststoffstrang 2 aus geeignetem Werkstoff, zum Beispiel Polyethylen, Polytetrafluorethylen oder Polyurethan, der der Blutkoagulation entgegenwirkt. Der Kunststoff kann Röntgenkontrastmittel enthalten. Der Versteifungskern 1 hat kreisförmigen Querschnitt und in seiner Mitte befindet sich eine rei.feste und rückstellfähige Seele, die in den Kunststoff eingebettet ist. Die Seele besteht aus einer geflochtenen oder gezwirnten Litze 3 aus einer Vielzahl von Monofilen 4. Bei dem gewählten Beispiel haben die Monofilen 4 zueinander gleichen Durchmesser. Die Litze 3 besteht aus Stahldrähten, so daß sie die Darstellung des Versteifungskerns 1 und damit des ihn umgebenden Katheterschlauches 5 auf dem Röntgenschirm verbessert. Außerdem verleiht die Litze 3 dem Versteifungskern 1 hohe Zugfestigkeit und Rückstellfähigkeit. Hierdurch wird ein AbreiDen des Versteifungskerns 1 bei sehr hohem Gehalt an Röntgenkontrastmittel vermieden und es werden dem Versteifungskern 1 gute Führungseigenschaften beim Einschieben in gekrümmte Körperhöhlen verliehen.

Die physikalischen Eigenschaften des Versteifungskerns 1 lassen sich zur Anpassung an gewünschte Einsatzzwecke beliebig variieren, indem der Durchmesser der einzelnen Monofile 4 und ihre Anzahl sowie die Art der für den Kunststoffstrang 2 verwendeten Kunststoffe verändert werden.

An einem Ende des Versteifungskernes 1 ist ein kappenartiges Griffstück 7 befestigt, dessen Außenfläche mit einer Rändelung 8 versehen ist. Im Gebrauchszustand überfängt das Griffstück 7 das Ende eines Katheteransatzes 9 lose, in dessen Stutzen 10 der Katheterschlauch 5 eingesetzt und befestigt ist. Ein Kragen 11 dient als Abrutschsicherung für eine Mutter 12 zur Kupplung des Katheteransatzes 9 mit einem Anschlußteil.

Bei Anbringung einer hochflexiblen Spitze am einführungsseitigen Ende des Versteifungskerns 1 kann dieser nicht nur als totraumfüllendes versteifendes Element für einen Katheterschlauch 5, sondern auch als Leitschiene für einen nach der sogenannten Seldinger-Methode in Körperhöhlen zu plazierenden Venenkatheter 6 benutzt werden. Der Versteifungskern 1 ist aufgrund seiner preiswerten Herstellbarkeit zum Einmalgebrauch geeignet.

## Patentansprüche

1. Katheterbesteck , bestehend aus einem flexiblen Katheterschlauch (5) mit zylindrischem Hohlraum und einem stabförmigen Versteifungskern (1), bei dem mindestens eine reißfeste Seele aus mehreren Drähten (4) in eine Umhüllung (2) aus Kunststoff eingebettet ist und der aus dem Hohlraum des Katheterschlauches (5) herausziehbar ist, dadurch gekennzeichnet, daß die reißfeste Seele als rückstellfähige Litze (3) aus geflochtenen oder gezwirnten Stahldrähten (4) ausgebildet ist und daß der Versteifungskern (I) den Hohlraum des Katheterschlauches (5) spaltfrei ausfüllt.

2. Katheterbesteck nach Anspruch 1, dadurch gekennzeichnet, daß die Litze (3) in der Umhüllung (2) konzentrisch angeordnet ist.

3. Katheterbesteck nach Anspruch 1 oder 2, dadurch gekenzeichnet, daß die Stahldrähte (4) der Litze (3) gleiche Stärke aufweisen.

4. Katheterbesteck nach Anspruch 1 oder 2, dadurch gekennzeichnet, daß die Stahldrähte der Litze zueinander verschiedene Stärke aufweisen.

5. Katheterbesteck nach einem der Ansprüche 1 bis 4, dadurch gekennzeichnet, daD die Litze (3) linksoder rechüsgeschlageh ist.

6. Katheterbesteck nach einem der Ansprüche 1 bis 5, dadurch gekennzeichnet, daß die Umhüllung (2) aus verschiedenen Kunststoffen besteht.

7. Katheterbesteck nach einem der Ansprüche 1 bis 6, dadurch gekennzeichnet, daß mit dem Einführende der Umhüllung (2) eine hochflexible Spitze aus Kunststoff fest verbunden ist.

8. Katheterbesteck nach Anspruch 7, dadurch gekennzeichnet, daß die Spitze hohl ausgebildet ist.

9. Katheterbesteck nach Anspruch 7, dadurch gekennzeichnet, daß die Spitze massiv ausgebildet ist.

## Claims

1. Catheter set comprising a flexible catheter tube (5) having a cylindrical lumen and a rod-shaped stiffening stem (1) in which at least one tear-resistant center formed by a plurality of wires (4) is embedded in a casing (2) of plastic and which is extractable from the lumen of the catheter hose (5), characterized in that the tear-resistant stem is a resilient strand (3) of braided or twisted steel wires (4) and that the stiffening core (1) provides a gap-free filling of the lumen of the catheter tube (5).

2. Catheter set as defined in claim 1, characterized in that the strand (3) is embedded concentrically- in the casing (2).

3. Catheter set as defined in claim 1 or 2, characterized in that the steel wires are of equal cross sectional area.

4. Catheter set as defined in claim 1 or 2, characterized in that the steel wires of the strand have mutually varying cross sectional areas.

5. Catheter set as defined in one of claims 1 to 4, characterized in that the lay of the strand (3) is left-hand or right-hand.

6. Catheter set as defined in one of claims 1 to 5, characterized in that the casing (2) consists of a plurality of plastic materials.

7. Catheter set as defined in one of claims 1 to 6, characterized in that a highly flexible tip of plastic is firmly connected to the insertion end of the casing (2).

8. Catheter set as defined in claim 7, characterized in that the tip is hollow.

9. Catheter set as defined in claim 7, characterized in that the tip is solid.

## Revendications

1. Ensemble de cathéter, constitué par un tuyau souple de cathéter (5) comportant une cavité cylindrique et un noyau raidisseur en forme de barreau (1), dans lequel au moins une âme résistante à la rupture et constituée par plusieurs fils (4) est insérée dans une gaine (2) en matière plastique et qui peut être retiré de la cavité du tuyau (5) du cathéter, caractérisé en ce que l'âme résistante à la rupture est réalisée sous la forme d'un cordon (3) présentant un effet de rappel et formé par des fils d'acier (4) tressés ou torsadés et que le noyau raidisseur (1) remplit la cavité du tuyau (5) du cathéter sans laisser subsister aucune fente.

2. Ensemble de cathéter selon la revendication 1, caractérisé en ce que le cordon (3) est disposé concentriquement dans la gaine (2).

3. Ensemble de cathéter selon la revendication 1 ou 2, caractérisé en ce que les fils d'acier (4) du cordon (3) présentent la même grosseur.

4. Ensemble de cathéter selon la revendication 1 ou 2, caractérisé en ce que les fils d'acier du cordon présentent des grosseurs différentes.

5. Ensemble de cathéter selon l'une des revendications 1 à 4, caractérisé en ce que le cordon (3) est enroulé dans le sens sinistrorsum ou dans le sens dextrorsum.

6. Ensemble de cathéter selon l'une des revendications 1 à 5, caractérisé en ce que la gaine (2) est constituée par différentes matières plastiques.

5. Ensemble de cathéter selon l'une des revendications 1 à 6, caractérisé en ce qu'une pointe très flexible en matière plastique est reliée rigidement â l'extrémité d'introduction de la gaine (2).

8. Ensemble de cathéter selon la revendication 7, caractérisé en ce que la pointe est creuse.

9. Ensemble de cathéter selon la revendication 7, caractérisé en ce que la pointe est pleine.

0 079 486

FIG. 1

FIG. 2

FIG. 3